# EUROPEAN PATENT APPLICATION

(11) **EP 4 674 369 A1**
(43) Date of publication of application: **07.01.2026**
(21) Application number: 24185792.9
(22) Date of filing: 01.07.2024
(51) Int. Cl.: A61B 34/00, A61B 90/00

(54) **METHOD AND SYSTEM FOR VISUAL SUPPORT OF A SURGICAL PROCEDURE, COMPUTER PROGRAM PRODUCT AND COMPUTER-READABLE MEDIUM**

(71) Applicant: OLYMPUS WINTER & IBE GMBH, 22045 Hamburg (DE)
(72) Inventor: ENDERS, Borg, 21149 Hamburg (DE); KUAN, Llin Ie Clarinda, 22393 Hamburg (DE)
(74) Representative: Seemann & Partner Patentanwälte mbB

(57) **Abstract**

The invention, inter alia, relates to a method for visual support of a surgical procedure as well as a system for visual support of a surgical procedure.

A method for visual support of an, in particular endoscopic or open, surgical procedure, wherein visual information of the surgical procedure is superimposed by at least one overlay containing additional, in particular surgical, information inside a field of view (20), wherein an image processor (56) controls a video display (52) to display the at least one overlay, comprises that the field of view (20) is divided into a region of interest (ROI) (22) and a peripheral region (24), wherein a transparency of each of the at least one overlay is determined depending on its position being located within the ROI (22) or the peripheral region (24).

## Description

The invention relates to a method for visual support of an, in particular endoscopic or open, surgical procedure. Furthermore, the invention concerns a system for visual support of a surgical procedure, a computer program product as well as a computer-readable medium.

With augmented and virtual reality becoming more and more common in applications across all parts of everyday life also overlay technologies become more common to enrich a field of vision with additional information.

To this end, artificial visual elements are merged with real life information either by overlaying this on top of natural vision using special glasses or by embedding overlays in video feeds that are recorded with camera systems. In the medical field, such a camera system may be an endoscope, laparoscope or microscope, a.o.

Typical overlays in a surgical context may comprise patient-specific 3D models of parts of the patient's anatomy or highlighting of identified structures within the area of surgery. Furthermore, information on equipment such as status or equipment settings as well as warnings for adverse events can be displayed as overlay.

It is known that in a single video feed multiple overlays with different information may be included.

Typically, overlays are defined with a transparency to allow for both the visibility of the overlay as well as the real life vision or video below the overlay.

Depending on the degree of transparency, on the one hand an overlay such as a patient-specific 3D model may hide underlying organic structures, causing the surgeon to miss details obscured by the overlay. On the other hand, a too transparent overlay may be difficult to read for the user. This causes extra workload to users to switch the transparency of overlays manually, in order to obtain conditions optimal for surgery.

It is an objective of the invention to improve the use of overlays in the context of displaying images of a surgical procedure on a video display.

The objective is solved by a method for visual support of an, in particular endoscopic or open, surgical procedure, wherein visual information of the surgical procedure is superimposed by at least one overlay containing additional, in particular surgical, information inside a field of view, wherein an image processor controls a video display to display the at least one overlay, wherein the field of view is divided into a region of interest (ROI) and a peripheral region, wherein a transparency of each of the at least one overlay is determined depending on its position being located within the ROI or the peripheral region.

A basic concept of the invention is to support a surgical procedure visually, wherein the at least one overlay has a transparency that depends on where it is located within the field of view. In particular, the method comprises that an image processor dynamically adapts the transparency of the at least one overlay. The transparency may be adapted with requirements linked to the surgical procedure, for example with regard to users' preferences and/or to varying circumstances of the surgical procedure or shifting of the ROI.

According to the invention, visual information of the surgical procedure is superimposed by at least one overlay containing additional information. Such visual information may comprise real-world optical vision, which is superimposed by at least one overlay by, e.g., extended reality glasses that comprise see-through glasses joined with a display for overlays. Alternatively, visual information may be images of the surgical procedure captured by a capturing device, wherein the images are displayed on a video display together with the overlays. In both cases the field of view describes the field of view for the surgeon.

For one surgical procedure, the regions of interest and the corresponding transparencies are not required to be constant. Each step, in particular each image, of the surgical procedure may be analyzed and the field of view for this step of the procedure may be divided into a region of interest and a peripheral region with different transparencies, depending on the user's interest to see the image content or the overlay.

Overlays contain additional, in particular surgical information. In particular, overlays in a surgical context may comprise patient-specific 3D models of parts of the patient's anatomy or may highlight identified structures within the visual information of the surgical procedure. E.g., an organ or a blood vessel may be detected and labelled with an overlay. Furthermore, information on equipment such as a status or equipment settings as well as warnings for adverse events can be displayed as overlay. Besides, surgical information contained by an overlay may be a subset of a patient's vital data, such as heart beat rate or blood pressure. Some overlays may be characterized as text with a background, wherein a transparency describes both the transparency of the text and the background, or may be characterized as augmented reality feature, depicting one-, two-, or three-dimensional objects.

In an embodiment, the transparency of each of the at least one overlay is higher in the ROI than in the peripheral region. In this manner, overlays in the peripheral region are easy to read, while in the ROI the images are the dominant visual contribution with rather fainter overlays. For example, the ROI may be located in the center of the field of view, where the main features of the surgery are usually shown. In this region, the surgeon may want to focus on the patient's tissue, and in the peripheral region, where the depicted tissue is not particularly important to the operation, the field of view may be covered with less transparent overlays more clearly showing surgical information.

A field of view with overlays can be adapted to different surgical procedures and provide clear depiction of the surgical procedure, if the ROI is a single area or if the ROI comprises multiple unconnected areas in the field of view. A single area may be used in open surgery, when in a simple embodiment of the method a center area of the field of view is chosen as ROI. Multiple unconnected areas as ROI may be useful, if in an open surgery multiple organs or other structures such as bleeding locations, cancerous growths or the like are visible in the ROI and all of them qualify as important to the procedure. In another example, multiple unconnected areas may relate to multiple blood vessels being highlighted within the field of view.

The least one overlay is particularly well-adapted to the surgical procedure, if the sizes and/or positions of the ROI and the peripheral region and/or the transparency for each overlay's position within the field of view is or are determined depending on procedure properties characterizing the surgical procedure, wherein, in particular, the procedure properties may comprise at least one of a procedure type, a procedure step, a surgeon, surgeon preferences, a used instrument and an event having occurred during procedure.

For example, for a given procedure type such as appendectomy it may be preferable to choose a different size of ROI than for a coronary artery bypass. For one procedure, a larger ROI may be chosen with respect to the other. Furthermore, for different procedure steps, e.g. preparatory, main, or final steps of the procedure, different areas of the field of view may be of importance for the surgeon. Thus, the sizes and/or positions of the ROI and the peripheral region may be determined according to the presently performed step of the procedure.

The same also holds for the transparency for each overlay's position within the field of view. While for some procedure types and/or procedure steps, the real world information is more important, such that transparency is set high, for other procedure properties, the transparency may be set low.

In embodiments, the ROI and the peripheral region and/or the transparency for each overlay's position is determined depending on the surgeon and/or surgeon preferences. For example, while for some users a large ROI is preferred, others may be accustomed to a smaller ROI. In particular, different surgeons may prefer different transparencies with respect to their eyesight. For example, color blindness may require higher or lower transparency of overlays, which may be included in the surgeon preferences for the procedure properties as well as possibly color or color contrast preferences.

Instruments in use may indicate predetermined sizes and/or positions of the ROI and the peripheral region and/or the transparency for each overlay's position within the field of view. For example, if an endoscope is used, the ROI may be chosen differently than for open surgery. Furthermore, a predetermined instrument may act as indicator for a procedure type or procedure step, wherein the ROI and the peripheral region and/or the transparency is adapted to.

For example, an event having occurred during a procedure may be one of a bleeding and active usage of an energy or dissection instrument. In case of such an event, the surgeon may require a different division between ROI and peripheral region and/or different values of transparency compared to the situation before the event occurs. For example, if a bleeding is detected, overlays comprising a 3D twin of an area of the patient's tissue may be increased in transparency, such that the surgeon is able to stop the bleeding before continuing with the planned procedure, for which the 3D overlay is needed.

Preferably, the procedure properties are, in particular at least partially, entered manually and/or loaded from a database.

Entering manually, in particular at least a subset of, procedure properties requires an additional step performed by staff. However, such input is very reliable. For example, the type of procedure as well as the name of the surgeon may be used as manual input to the image processor. The manual input may include typing and/or selecting from a list of possible entries, such as surgeon's names or predefined procedures. Depending on these manually entered procedure properties, e.g., the sizes and/or positions of the ROI and the peripheral region are determined.

Alternatively, procedure properties may be loaded from a database. For example, using an ID for the surgery procedure, a list of procedure properties may be obtained automatically from a database. This may comprise, e.g., used instruments and procedure type.

The method may be further developed in that a capturing device, in particular a video endoscope or a camera, captures images of the surgical procedure within the field of view, wherein the image processor receives the captured images from the capturing device and processes the images of the surgical procedure. This enhances further the visual support of the surgical procedure.

In an embodiment, the image processor controls the video display to display the images of the surgical procedure superimposed with the at least one overlay. This way, the visual information of the surgical procedure being superimposed with at least one overlay are the images being displayed on the video display. For example, this is implemented by using a screen and/or virtual reality glasses as video display. This allows for precise alignment of overlays within the field of view.

Furthermore, the images captured by the capturing device may improve the visual support of the surgical procedure, if the procedure properties are, in particular at least partially, automatically detected by the image processor from the images of the surgical procedure, in particular by means of a neural network and/or machine learning methods.

For example, the image processor may be configured to analyze the images of the surgical procedure and automatically detect, which procedure type or procedure step is performed. According to this obtained procedure property, the sizes and/or positions of the ROI and the peripheral region and/or the transparency of the overlays is dynamically adapted. In particular, events having occurred, such as bleeding or usage of a predetermined instrument, may be detected by image analysis.

Additionally or alternatively, the image processor may automatically detect procedure properties using information provided by other devices, such as a surgery information management system or a capturing device such as an endoscope. Such other devices may supply information, such as used instruments or users or planned procedure type, to the image processor.

In an embodiment, the sizes and/or positions of the ROI and the peripheral region and/or the transparency for each overlay's position within the field of view is or are loaded from a database, depending on the procedure properties characterizing the surgical procedure. For different procedure properties, predetermined values for the ROI and/or transparency may be stored in the database, which are loaded when the corresponding procedure is identified to take place.

Automatic detection of procedure properties by the image processor is in particular achieved by means of a, in particular pre-trained and/or trained, neural network and/or machine learning methods. In particular, a neural network is trained by a multitude of images of surgical procedures, in order to recognize structures and relate them to procedure properties.

The method for visual support of a surgical procedure is particularly easy to implement, if the transparency of each of the at least one overlay assumes one of two values, wherein one value is assigned to overlays within the ROI and the other value is assigned to overlays in the peripheral region. With exactly one value for each region it is not necessary for each overlay to identify its exact position. Instead, it suffices to assign each overlay either to the ROI or the peripheral region, in order to determine its transparency.

In an alternative embodiment, the transparency of each of the at least one overlay assumes a constant value within the ROI and/or the transparency of each of the at least one overlay decreases within the peripheral region with increasing distance from the ROI. For example, the transparency within the peripheral region may decrease linearly with increasing distance from the ROI. This allows for a more differentiated display of overlays without a strong cut-off of transparencies at the border between ROI and peripheral region.

In another embodiment, the transparency of each of the at least one overlay decreases within both the ROI and the peripheral region with increasing distance from the center of the ROI. In particular, this way to determine the transparency is suitable for surgical procedures where the ROI is a single area, in particular if a small center is most important to be viewed by surgical staff.

Visual support of a surgical procedure using transparent overlays is further improved, if at least a part of the ROI is identified by detecting a predetermined structure within the field of view, in particular a tissue and/or a surgical instrument, wherein the size and/or position of the ROI is dynamically adjusted, when the predetermined structure moves within the field of view. This allows for the overlays' transparency to be adjusted automatically, sustaining an optimal view for the surgeon. For example, a blood vessel may be detected by the image processor and the ROI set at the position of the blood vessel with a margin of 5 millimeter around the blood vessel. In another example, an organ, such as a liver, may be detected and the area covered by the organ chosen as ROI together with a margin of, e.g., 10 millimeters or 20 millimeters.

Detecting an event having occurred, such as a bleeding or active usage of a predetermined instrument, may be used as a trigger to add an additional ROI, e.g. in the area of bleeding or usage of the instrument, and/or to change the transparency of the overlays.

The object underlying the invention is also solved by a system for visual support of a surgical procedure comprising a video display and an image processor, wherein the image processor is configured to perform a method for visual support of a surgical procedure as described above.

The system has the same technical effects and the same advantages as the method described above, to which specific reference is made.

In particular, the video display comprises a screen and/or extended reality glasses. In an embodiment, the images of the surgical procedure are shown on a screen, wherein the images are superimposed with overlays showing additional, in particular surgical, information.

In such an embodiment, the images are visual reproductions of acquired video data.

Furthermore, the images of the surgical procedure may be shown using extended reality glasses. This includes both extended reality glasses which transmit real world optical information to the human eye, such as regular glasses, adding further information such as overlays, as well as virtual reality glasses that reproduce the optical real world information on a nontransparent head-mounted display and add at least one overlay.

In an embodiment, the system comprises a capturing device, in particular at least one of a camera, an endoscope, a laparoscope and a microscope. Such capturing devices are well-known for providing high-quality images of a surgical procedure. Alternatively, e.g. when the system comprises extended reality glasses that allow for natural vision through transparent glasses, a capturing device, in particular a camera, may be comprised in such extended reality glasses. For example, in the latter case, the captured images are not shown on a display, yet may be used for determining procedure properties or for aligning the overlays with the field of view of the surgeon wearing the extended reality glasses.

Furthermore, the object is solved by a computer program product comprising instructions which, when the program is executed by a computer, cause the computer to carry out the method as described above.

Additionally, the object is solved by a computer-readable medium having stored thereon the above-mentioned computer program product.

Both have the same technical effects and the same advantages as the method and the display system described above, to which specific reference is made.

Moreover, the object is solved by a method of performing an, in particular endoscopic or open, surgical procedure, wherein visual information of the surgical procedure is superimposed by at least one overlay containing additional, in particular surgical, information inside a field of view, wherein an image processor controls a video display to display the at least one overlay, characterized in that the field of view is divided into a region of interest (ROI) and a peripheral region, wherein a transparency of each of the at least one overlay is determined depending on its position being located within the ROI or the peripheral region.

In particular, the method of performing a surgical procedure comprises features of the above-described method of visual support of a surgical procedure and/or is carried out using the above-described visual support system.

Further characteristics of the invention will become apparent from the description of the embodiments according to the invention together with the claims and the included drawings. Embodiments according to the invention can fulfill individual characteristics or a combination of several characteristics.

The invention is described below, without restricting the general intent of the invention, based on exemplary embodiments, wherein reference is made expressly to the drawings with regard to the disclosure of all details according to the invention that are not explained in greater detail in the text. The drawings show in:
- Fig. 1: a first embodiment of a field of view with a region of interest,
- Fig. 2: a second embodiment of a field of view with a region of interest,
- Fig. 3: a third embodiment of a field of view with a region of interest,
- Fig. 4: a flowchart of a first embodiment of a method for visual support of a surgical procedure,
- Fig. 5: a flowchart of a second embodiment of a method for visual support of a surgical procedure,
- Fig. 6: an embodiment of a system for visual support of a surgical procedure,
- Fig. 7: a schematic representation of a computer program product, and
- Fig. 8: a schematic representation of a computer-readable medium.

In the drawings, the same or similar types of elements or respectively corresponding parts are provided with the same reference numbers in order to prevent the item from needing to be reintroduced.

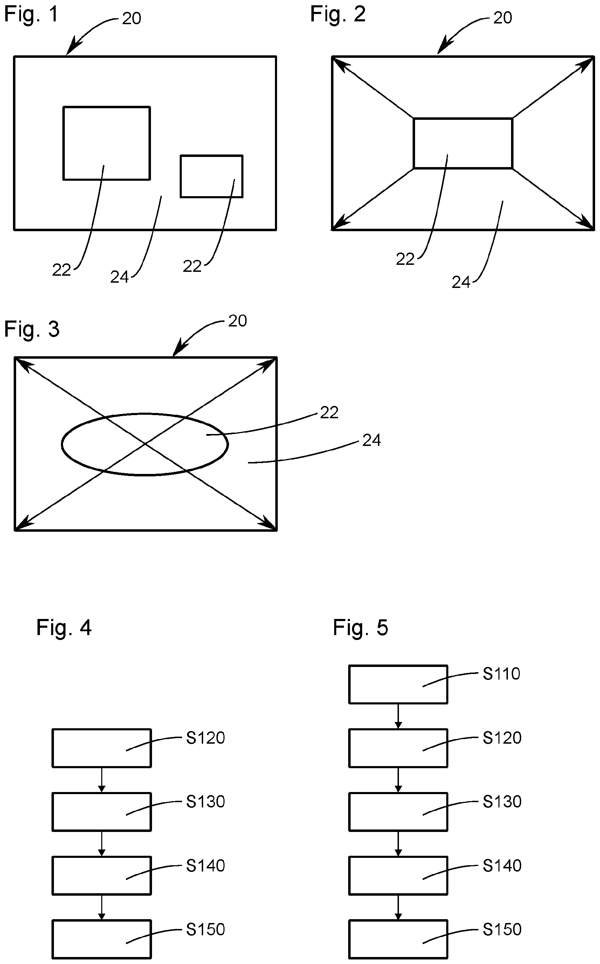

Figures 1 to 3 show three different embodiments of a field of view 20 with different sizes and positions of an ROI 22 and the peripheral region 24, with different transparency values for overlays within the field of view 20, depending on their positions within or without the ROI 22.

Fig. 1 depicts a rectangular field of view 20 with an ROI 22 comprising two off-center, unconnected areas being surrounded by a peripheral region 24. In this embodiment, the transparency of each overlay within the ROIs 22 assumes a first value and the transparency of each overlay within the peripheral region 24 assumes a second value, typically lower than the first value.

Fig. 2 shows another embodiment for a field of view 20 with ROI 22 located in its center. Any overlay within the ROI 22 is assigned a constant transparency. For overlays within the peripheral region 24 surrounding the ROI 22, the overlays' transparency decreases with increasing distance from the ROI. Hence, the further to the rim of the field of view 20, the more opaque an overlay will be displayed.

Fig. 3 depicts an oval shaped ROI 22 within a rectangular field of view 20. In this embodiment, the transparency of overlay's decreases with distance from the center of the field of view 20. Hence, within both the ROI 22 and the peripheral region 24 the transparency of overlays is not constant and depends on the position within the field of view 20.

Typical overlays to be located at the rim of the field of view 20 may be the status of equipment, such as surgical instruments, and/or equipment settings. Furthermore, patient's vital data may be shown as overlay in the outer part of the peripheral region 24. Typical overlays shown in the ROI 22 or close to the ROI 22 within the peripheral region 24 are patient-specific 3D models of parts of the patient's anatomy.

Fig. 4 sketches a flowchart of a first embodiment of a method for visual support of a surgical procedure. In a first step, procedure properties of the surgical procedure are determined (S120). In this example, the procedure type and the used instruments for the procedure are entered manually by a user, such as the surgeon or support staff. In a second step, the sizes and positions of the ROI 22 and the peripheral region 24 are determined (S130). In this embodiment, the image processor 56 retrieves the sizes and positions from a database as response to an input of the procedure properties. In a similar fashion, the transparencies for the ROI 22 and the peripheral region 24 are determined by the image processor 56 (step S140). Finally, the image processor 56 superimposes visual information by at least one overlay containing additional surgical information (step S150), using the predetermined settings for sizes and positions of ROI 22 and peripheral region 24 as well as the transparencies.

The method may be executed using extended reality glasses that allow to superimpose transparent overlays with real-world visual data through see-through glasses. The parameters for the regions within the field of view 20 as well as the transparencies are determined in the beginning of the surgical procedure. Subsequently, overlays may be shown and/or turned off, yet always using the same set of parameters. Alternatively, the overlays may be superimposed on a video stream and shown on a screen.

Fig. 5 shows a flowchart of a second embodiment of a method for visual support of a surgical procedure. In this embodiment, the depiction of overlays, including their transparency, is adapted dynamically during the surgical procedure. Therefore, the steps described in the following may be performed repeatedly.

In a first step, a capturing device 58 captures images of the surgical procedure within the field of view (step S110). The capturing device 58 may be a video endoscope 59. The image processor 56 receives the captured images from the capturing device 58 and processes the images of the surgical procedure. The image processor 56 uses the images to determine procedure properties (step S120). For example, by image recognition and machine learning techniques, the image processor 56 identifies the procedure type and the procedure step of the surgical procedure. For example, it is identified, if it is the beginning, the main phase, or the final phase of a predetermined procedure. The image recognition and machine learning model will have been trained with training videos and/or images of different types of procedures in different stages that may have been labelled accordingly for training purposes.

In embodiments of the method, the procedure properties are determined jointly by manual input of the user as well as automatic detection by the image processor 56. For example, while the procedure type may be entered manually, the procedure step may be detected automatically from the captured images. Besides, while information about a surgeon may be entered manually, used instruments may be detected automatically.

Furthermore, the image processor 56 may be programmed or trained to recognize surgical instruments within the field of view 20 and to recognize their usage as procedure property. In particular, the image processor 56 may be triggered to search for specific surgical instruments, wherein the specific surgical instrument are either being manually predetermined in a list of selected instruments for the surgical procedure or are saved in a database as being common for a predetermined procedure type.

Depending on the determined procedure properties, the sizes and positions of the ROI 22 and the peripheral region 24 as well as the corresponding transparencies are determined (steps S130, S140). For example, for predetermined properties, the image processor 56 loads predetermined values for sizes, positions and/or transparencies from a database. In particular, for each procedure step of a given procedure type, a different set of parameters for sizes, positions and/or transparencies is stored.

In a final step S150, the image processor 56 superimposes visual information of the surgical procedure by at least one overlay using the parameters determined in the previous steps S130, S140.

In this embodiment, the method is performed using a video endoscope 59 for capturing images of the surgical procedure. These images are displayed as visual information of the surgical procedure on a video display 52. Furthermore, the image processor 56 superimposes the overlays on the same video display 52.

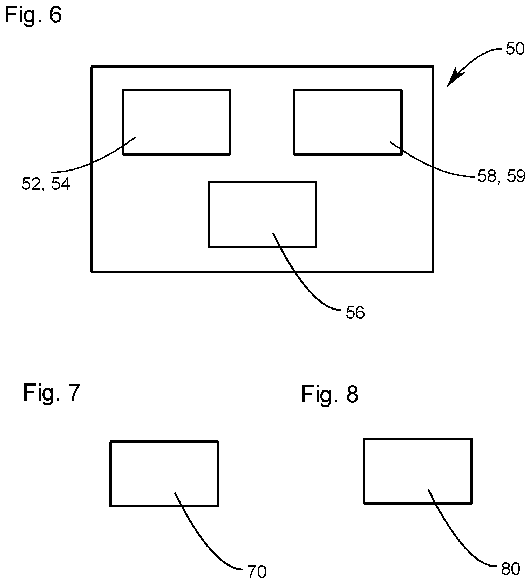

Fig. 6 shows an embodiment of a system 50 for visual support of a surgical procedure comprising an image processor 56 and a video display 52. In this embodiment, the video display 52 may be a screen 54. Furthermore, the system 50 comprises a capturing device 58, for example an endoscope 59. Jointly, the system 50 is configured to perform a method for visual support of a surgical procedure as described above.

Fig. 7 represents schematically a computer program product 70 comprising instructions which, when the program is executed by a computer, cause the computer to carry out a method for visual support of a surgical procedure as described above.

Fig. 8 represents schematically a computer-readable medium 80 having stored thereon a computer program product as depicted in Fig. 7.

All named characteristics, including those taken from the drawings alone, and individual characteristics, which are disclosed in combination with other characteristics, are considered alone and in combination as important to the invention. Embodiments according to the invention can be fulfilled through individual characteristics or a combination of several characteristics. Features which are combined with the wording "in particular" or "especially" are to be treated as preferred embodiments.

### List of References

- 20: field of view
- 22: region of interest (ROI)
- 24: peripheral region
- 50: system for visual support of a surgical procedure
- 52: video display
- 54: screen
- 56: image processor
- 58: capturing device
- 59: endoscope
- 70: computer program product
- 80: computer-readable medium

- S110: capturing images of the surgical procedure
- S120: determining procedure properties
- S130: determining sizes and positions of ROI and peripheral region
- S140: determining transparencies
- S150: superimposing visual information with at least one overlay

## Claims

1. Method for visual support of an, in particular endoscopic or open, surgical procedure, wherein visual information of the surgical procedure is superimposed by at least one overlay containing additional, in particular surgical, information inside a field of view (20), wherein an image processor (56) controls a video display (52) to display the at least one overlay, **characterized in that** the field of view (20) is divided into a region of interest (ROI) (22) and a peripheral region (24), wherein a transparency of each of the at least one overlay is determined depending on its position being located within the ROI (22) or the peripheral region (24).

2. Method according to claim 1, wherein the transparency of each of the at least one overlay is higher in the ROI (22) than in the peripheral region (24).

3. Method according to claim 1 or 2, wherein the ROI (22) is a single area or the ROI (22) comprises multiple unconnected areas in the field of view (20).

4. Method according to one of the claims 1 to 3, wherein the sizes and/or positions of the ROI (22) and the peripheral region (24) and/or the transparency for each overlay's position within the field of view (20) is or are determined depending on procedure properties characterizing the surgical procedure,
wherein in particular the procedure properties comprise at least one of a procedure type, a procedure step, a surgeon, surgeon preferences, a used instrument and an event having occurred during procedure.

5. Method according to claim 4, wherein the procedure properties are, in particular at least partially, manually entered and/or loaded from a database.

6. Method according to claims 1 to 5, wherein a capturing device (58), in particular a video endoscope or a camera, captures images of the surgical procedure within the field of view (20), wherein the image processor (56) receives the captured images from the capturing device (58) and processes the images of the surgical procedure.

7. Method according to claim 6, wherein the image processor (56) controls the video display (52) to display the images of the surgical procedure superimposed with the at least one overlay.

8. Method according to claim 6 or 7, wherein the procedure properties are, in particular at least partially, automatically detected by the image processor (56) from the images of the surgical procedure, in particular by means of a neural network and/or machine learning methods.

9. Method according to one of the claims 1 to 8, wherein the transparency of each of the at least one overlay assumes one of two values, wherein one value is assigned to overlays within the ROI (22) and the other value is assigned to overlays in the peripheral region (24), or
the transparency of each of the at least one overlay assumes a constant value within the ROI (22) and/or the transparency of each of the at least one overlay decreases within the peripheral region (24) with increasing distance from the ROI (22).

10. Method according to one of the claims 1 to 9, wherein at least a part of the ROI (22) is identified by detecting a predetermined structure within the field of view, in particular a tissue and/or a surgical instrument, wherein the size and/or position of the ROI (22) is dynamically adjusted, when the predetermined structure moves within the field of view.

11. System (50) for visual support of a surgical procedure comprising a video display (52) and an image processor (56), wherein the image processor (56) is configured to perform a method for visual support of a surgical procedure according to one of the claims 1 to 10.

12. System (50) according to claim 11, wherein the video display (52) comprises a screen (54) and/or extended reality glasses.

13. System (50) according to claim 11 or 12, the system (50) further comprising a capturing device (58), in particular at least one of a camera, an endoscope (59), a laparoscope and a microscope.

14. Computer program product (70) comprising instructions which, when the program is executed by a computer, cause the computer to carry out the method of one of the claims 1 to 10.

15. A computer-readable medium (80) having stored thereon the computer program product (70) of claim 14.
